# EUROPEAN PATENT APPLICATION

(11) **EP 2 468 363 A2**
(43) Date of publication of application: **27.06.2012**
(21) Application number: 11179948.2
(22) Date of filing: 02.09.2011
(51) Int. Cl.: A61Q 19/00, A61Q 19/08, A61K 8/02, A61K 8/34, A61K 8/37, A61K 8/86, A61K 8/893, A61K 8/894, A61K 8/898

(54) **Compositions comprising a silicone water-in-oil emulsifier and a low nitrogen containing amino-functional silicone gum**

(30) Priority: 02.09.2010 US 874342
(71) Applicant: Johnson & Johnson Consumer Companies, Inc., Skillman, NJ 08558 (US)
(72) Inventor: Narasimhan, Saroja, Monmouth Junction, NJ New Jersey 08852 (US); Librizzi, Joseph J., Hillsborough, NJ New Jersey 08844 (US)
(74) Representative: Kirsch, Susan Edith

(57) **Abstract**

Provided are compositions comprising a silicone water-in-oil emulsifier, a low nitrogen containing amino-functional silicone gum, a hydrophobic agent, a non-silicone primary emulsifier, and water.

## Description

### FIELD OF INVENTION

The present invention relates to compositions comprising a silicone emulsifier and an amino-functional silicone gum, methods of making such compositions and uses of such compositions in personal care products.

### DESCRIPTION OF THE RELATED ART

A variety of personal care compositions are available to provide various skin care benefits. It is important that the product have a desirable feel when applied. Organopolysiloxanes (i.e., silicones) have gained acceptance in skin-care formulations to aid in emolliency, spreading, and general enhancement of skin feel. In particular, so-called "silicone elastomers," polymers of organosiloxanes that have been crosslinked, in combination with silicone fluids, have been used in consumer products to impart an improved skin feel. Silicone elastomers and silicone fluids are sometimes described as providing a less heavy or less greasy feel compared to hydrocarbon-based emollients. However, silicone elastomers suffer from several drawbacks. These materials are difficult to formulate into personal care compositions in a way that allows for good shelf stability. Furthermore, silicone elastomers are expensive, and since one must generally use relatively high concentrations of typical silicone elastomers as well as high levels of silicone fluids along with the silicone elastomer to achieve the desired end feel, cost-effectiveness can be lacking. In addition, the tactile character that is imparted by silicone elastomers can be somewhat unpredictable, and dependent upon other ingredients that are used in the formulation.

In light of the above, applicants have recognized a need exists for a personal care composition providing more cost-effective, aesthetic and, in certain cases, more predictable skin feel benefits.

### SUMMARY OF THE INVENTION

The present invention meets the aforementioned need and overcomes the disadvantages of the prior art. In particular, applicants have discovered that one or more silicone water-in-oil emulsifier, can be combined with one or more low nitrogen containing amino-functional silicone gums, a non-silicone primary emulsifier, and a hydrophobic agent to produce compositions having a significant and unexpected skin-feel properties.

According to one aspect, the present invention provides a composition comprising water, a silicone water-in-oil emulsifier, an amino-functional silicone gum having a nitrogen content that is less than about 3%, a non-silicone primary emulsifier, and a hydrophobic agent.

According to another aspect, the present invention provides methods of treating the skin comprising topically applying to the skin a composition comprising water, a hydrophobic agent, a non-silicone primary emulsifier, a silicone water-in-oil emulsifier; and an amino-functional silicone gum having a nitrogen content that is less than about 3%.

### DESCRIPTION OF PREFERRED EMBODIMENTS

All percentages listed in this specification are percentages by weight, unless otherwise specifically mentioned.

As described above, applicants have found that compositions including a silicone water-in-oil emulsifier, a low nitrogen containing amino-functional silicone gum, a non-silicone primary emulsifier, and a hydrophobic agent can be combined to prepare compositions having desirable aesthetic/skin-feel properties. For example, as shown in the Examples, applicants have tested the "pickup" from a container associated with compositions of the present invention as compared to comparable compositions comprising either a silicone water-in-oil emulsifier or a low nitrogen containing amino-functional silicone gum (but not both). As illustrated, applicants have discovered that the claimed invention exhibits significant "pickup" as compared to the other compositions. Accordingly, the present compositions are particularly suitable for use in topical skin care products including, but not limited to, moisturizers, anti-aging products, anti-acne products, and the life.

Applicants have also discovered unexpectedly that, compositions of the present invention may include one or more liquid crystal (LC) phases distributed in an exterior phase. By "liquid crystal phase," it is meant a phase that exhibits positional order typical of a crystalline solid yet has attributes (such as flow) that are similar to liquids. The LC phase may include a lamellar phase. By "lamellar phase," it is meant sheet-like structures of hydrophobic moieties essentially sandwiched between hydrophilic moieties. The sheet like structures may be flat or take on curvature. The lamella may preferably take on curvature and form enclosed structures or vesicles. The vesicle may be essentially spherulitic, i.e., such as spherulites. The lamella may optionally arrange themselves into "multilamellar vesicles," a series of enclosed structures that are concentric or otherwise enclose one another. Suitably, the composition may include "worm-like" structures which are essentially structures that are "hybrid" between lamellar sheets and spherulites.

In general, unless otherwise specified, "hydrophobic moieties" may be taken to refer to moieties such as those (a) having a carbon chain of at least six carbons in which none of the six carbons is a carbonyl carbon or has a hydrophilic moiety (defined below) bonded directly to it; (b) having two or more alkyl siloxy groups; or (c) having two or more oxypropylene groups in sequence. Suitably, the hydrophobic moiety may include linear, cyclic, aromatic, saturated or unsaturated groups. The hydrophobic compound is preferably not amphiphilic and, as such, does not include hydrophilic moieties.

Unless otherwise specified "hydrophilic moieties" may be taken to refer to moieties such as anionic, cationic, zwitterionic, or nonionic group, that is polar, including sulfate, sulfonate, carboxylate, phosphate, phosphonates, ammonium, including mono-, di-, and trialkylammonium species, pyridinium, imidazolinium, amidinium, poly(ethyleimininium), ammonioalkylsulfonate, ammonioalkylcarboxylate, amphoacetate, and poly(ethyleneoxy)sulfonyl moieties.

Applicants have further discovered that compositions of the present invention are preferably structured so as to have long-range ordering as well as short-range ordering. By "short range ordering" it is meant that the liquid crystal phase exhibits orientational or positional order on the order that is typically on the molecular scale, such as less than about 100 Angstroms, and is typically "intra-particle," i.e., within particles such as spherulite particles. By "long-range ordering" it is meant that the liquid crystal phase exhibits order that is greater than about 100 Angstroms, preferably greater than about 150 Angstroms, more preferably greater than about 200 Angstroms, most preferably from about 200 Angstroms to about 1000 Angstroms. Long-range ordering is typical of "inter-particle" (i.e., between particles such as between spherulites). The composition preferably includes one or more liquid crystal phases having both short range ordering and long-range ordering. As would be readily understood by one skilled in the art, the scale of ordering in a composition and therefore the presence of long and short range ordering can be determined by small angle x-ray scattering (SAXS) as described in Example IV below.

Any of a variety of suitable silicone water-in-oil emulsifiers may be used in the compositions of the present invention. By "silicone" it is meant a molecule that includes at least one siloxane (-Si-O-) repeat unit and further includes a hydrophobic moiety and a hydrophilic moiety. By "water-in-oil emulsifier" it is meant a compound that when combined with deionized water and dimethicone in the following amounts (weight percent of total mixture on an active basis): 1% water-in-oil emulsifier, 96% dimethicone, and 3% deionized water, the resulting mixture can be agitated to form an emulsion of water in dimethicone that remains stable (no visible phase separation) for at least 24 hours when held at about 25°C.

The HLB3 (hydrophile-lipophile balance) of the silicone water-in-oil emulsifier is preferably relatively low. For example, the silicone emulsifier may have an HLB that is less than about 14, preferably from about 2 to about 13, more preferably from about 2 to about 10, most preferable from about 2 to about 9. For embodiments of the present invention comprising a combination of two or more silicone water-in-oil emulsifiers, the HLB of the overall combined silicone water-in-oil emulsifier is preferably as defined above. The HLB System was introduced in the late 1940`s by ICI Americas Inc. Methods of determining HLB are well known in the art and any of such methods may be used for HLB determination. A description of the HLB System and methods for HLB determination are described in "The HLB System: a time saving guide to emulsifiers selection," ICI Americas Inc. (ICI Surfactants); Wilmington, Delaware; 1976, 1984, 1992, page 19.

Examples of suitable silicone water-in-oil emulsifiers include, but are not limited to, (1) non-crosslinked dimethicone copolyols such as: alkoxy dimethicone copolyols, silicones having pendant hydrophilic moieties such as linear silicones having pendant polyether groups, branched polyether and alkyl modified silicones, branched polyglycerin and alkyl modified silicones; (2) crosslinked elastomeric solid organopolysiloxanes comprising at least one hydrophilic moiety; and combinations of two or more thereof, and the like. A variety of commercially available non-crosslinked dimethicone copolyols include the following mixtures sold by Dow Corning of Midland, Michigan: cyclopentasiloxane (and) PEG/PPG-18/18 Dimethicone available as DC 5225C, and cyclopentasiloxane (and) PEG-12 Dimethicone crosspolymer available as DC9011. Certain preferred non-crosslinked dimethicone copolyols are cetyl dimethicone copolyols such as Cetyl PEG/PPG-10/1 Dimethicone sold under the name Abil EM-90, branched polyether and alkyl modified silicones such as Lauryl PEG-9 Polydimethylsiloxyethyl Dimethicone sold under the name KF-6038, and branched polyglycerin and alkyl modified silicones such as Lauryl Polyglyceryl-3 Polydimethylsiloxyethyl Dimethicone sold under the name KF-6105. Other preferred non-crosslinked dimethicone copolyols include, for example, bis-PEG/PPG-14/dimethicone copolyol sold under the name Abil EM-97 and the polyglyceryl-4 isostearate/cetyl dimethicone copolyol/hexyl laurate mixture sold under the name Abil WE 09. Another suitable non- crosslinked dimethicone copolyol is PEG-9 Polydimethylsiloxyethyl Dimethicone sold under the name KF-6028. Abil EM-90, Abil EM-97 and Abil WE 09 are available from Evonik Goldschmidt GmbH of Essen, Germany. KF-6038 are KF-6105 are available from Shin-Etsu Silicones of Akron, Ohio.

By "crosslinked elastomeric solid organopolysiloxanes comprising at least one hydrophilic moiety," it is meant chemically or physically crosslinked molecules having at least one siloxane repeat unit and at least one hydrophilic moiety, wherein the material is generally flexible and deformable and having a modulus of elasticity such that the material is resistant to deformation and has a limited ability to expand and to contract. The material is capable of returning to its original shape after it has been stretched. This elastomer is formed of polymeric chains of high molecular weight, the mobility of which is limited by a uniform network of crosslinking points. The preferred crosslinked elastomeric solid organopolysiloxanes useful in the composition of the invention includes one or more oxyalkylenated groups as the hydrophilic moiety, and preferably oxyethylenated (OE) groups, for example from 1 to 40 oxyalkylene units and better still 1 to 20 oxyalkylene units, which can form polyoxyalkylene chains and in particular polyoxyethylene chains. The hydrophilic moiety may optionally be a hydroxyl group (or a plurality of hydroxyl groups, such as a polyglycerin). These groups can be pendant, at the chain end or intended to bond two parts of the silicone structures. The silicon atoms carrying these groups preferably number from approximately 1 to 10. These organopolysiloxanes can be provided in the form of a powder, the particles constituting this powder having a size ranging from 0.1 to 500 µm and better still from 3 to 200 µm and being able to be spherical, flat or amorphous with preferably a spherical shape. They can also be provided in the form of a gel comprising the elastomeric organopolysiloxane dispersed in an oily phase. This oily phase, also known as liquid fatty phase, can comprise any non-aqueous substance or mixture of non-aqueous substances which is liquid at room temperature (25° C.). The organopolysiloxanes of the invention may be obtained according to the procedure of Examples 3, 4 and 8 of U.S. Pat. No. 5,412,004 and Examples 1, 2 and 3 of U.S. Pat. No. 5,811,487, both incorporated herein in their entirety. Examples of suitable elastomeric organopolysiloxanes which can be used in the composition of the invention include polyether-modified crosslinked siloxanes such as Dimethicone/PEG-10/15 Crosspolymer (available from Shin-Etsu Silicones as KSG-210) and the like, or polyglycerin-modifed crosslinked siloxanes such as Dimethicotic/Polyglycerin-3 Copolymer (available from Shin-Etsu Silicones as KSG-710) and the like.

Any suitable amount of silicone water-in-oil emulsifier may be used in the compositions of the present invention. The compositions may preferably comprise silicone water-in-oil emulsifier in an amount of from about 0.1% to about 10% by weight of active emulsifier. More preferably, the compositions comprise from about 0.3% to about 5%, more preferably from about 0.4% to about 2.5%, more preferably from about 0.75% to about 1.5%.

Any of a variety of suitable amino-functional silicone gums may be used in accord with the present invention. By "amino-functional silicone gum," it is meant a gum molecule that includes at least one siloxane (-Si-O-) repeat unit and further includes at least one amine functionality, and is generally insoluble in water. The substitution of the amine may be primary, secondary, tertiary, or quaternary. The amino-functional silicone gum may be of any suitable molecular weight. The molecular weight is preferably from about 1000 to about 100,000.

As noted above, the inventors have found that surprisingly when the amino-functional silicone gum is selected to have a nitrogen content (i.e., a weight percent of nitrogen in the amino-functional silicone gum) that is less than about 3% the compositions of the present invention have excellent tactile aesthetics and also tend to favor the formation of long range ordering and short range ordering. Accordingly, the amino-functional silicone gum preferably has a weight percent of nitrogen therein (based on total weight of the gum) about 3% or less. The amino-functional silicone gum may have a weight percent of nitrogen of preferably from about 0.2 % to about 3%, more preferably from about 0.5% to about 3%, and even more preferably from about 0.75% to about 3%,

Examples of suitable amino-functional silicone gums having a nitrogen content of less than about 3% include certain aminoalkyldimethicones, e.g. primary amine-types such as KF 8018 from Shin-Etsu, which is an amino-modified silicone gum aminopropyldimethicone (nitrogen content of about 1%) 10% active dispersed in cyclopentasiloxane. Other suitable amino-functional silicone gums include morpholine or diethanolamine-modified polydimethylsiloxanes, e.g. tertiary amine-types such as Silamine Di-50-M, a morpholine modified polydimethylsiloxane (nitrogen content of about 0.8%) available from SilTech Corporation of Toronto, Canada.

Any suitable amount of amine-functional silicone gum may be used in the compositions of the present invention. The compositions may preferably comprise amine-functional silicone gum in an amount from about 0.1% to about 10%, preferably from about 0.5% to about 5%, more preferably from about 1.0% to about 5.0% by weight, even more preferably from about 1.5% to about 4.0% by weight.

Compositions of the present invention further include one or more hydrophobic agents. By "hydrophobic agents," it is meant a compound that is generally insoluble in water and includes a hydrophobic moiety, such as one meeting one or more of the following three criteria:
(a) has a carbon chain of at least six carbons in which none of the six carbons is a carbonyl carbon or has a hydrophilic moiety (defined below) bonded directly to it; (b) has two or more alkyl siloxy groups; or (c) has two or more oxypropylene groups in sequence. The hydrophobic moiety may include linear, cyclic, aromatic, saturated or unsaturated groups. The hydrophobic compound is preferably not amphiphilic and, as such, does not include hydrophilic moieties, such as anionic, cationic, zwitterionic, or nonionic group, that is polar, including sulfate, sulfonate, carboxylate, phosphate, phosphonates, ammonium, including mono-, di-, and trialkylammonium species, pyridinium, imidazolinium, amidinium, poly(ethyleneiminium), ammonioalkylsulfonate, ammonioalkylcarboxylate, amphoacetate, and poly(ethyleneoxy)sulfonyl moieties. Suitably, the hydrophobic agent does not include hydroxyl moieties.

The hydrophobic agent may be an oil. As used herein, "oil" means a hydrophobic agent that has a melting point that is below 30°C. Suitable examples of oils component include vegetable oils (glyceryl esters of fatty acids, triglycerides) and fatty esters. Specific nonlimiting examples include, without limitation, esters such as isopropyl palmitate, isopropyl myristate, isononyl isonanoate (such as WICKENOL 151 available from Alzo Inc. of Sayreville, NJ), C₁₂-C₁₅ alkyl benzoates (such as FINSOLV TN available from Finetex Inc.), caprylic/capric triglycerides. Suitably, the oil may be a silicone oil. By silicone oil it is meant an oil that includes at least one siloxane linkage. Suitable examples include Dimethicone (e.g., Dow Corning 200 Fluid, Dow Corning 1413 Fluid, both available from Dow Corning of Midland, Michigan) cyclopentasiloxane (e.g., Dow Corning 245 Fluid). Suitably, the oil may be mineral oil.

Other hydrophobic compounds suitable for use as hydrophobic agents in the continuous oil phase include high melting point hydrophobic compounds that do not necessarily meet the above definition of an "oil." Suitable examples include hydrophobic and/ or silicone compounds having a melting point that is above 30°C, including any of various silicone waxes or waxy hydrocarbons (straight or branched chain alkanes or alkenes, ketone, diketone, primary or secondary alcohols, aldehydes, sterol esters, alkanoic acids, turpenes, monoesters), such as those having a carbon chain length ranging from C₁₂-C₃₈. Examples of other suitable high melting point hydrophobic compounds include esters of alcohol (glycerol or other than glycerol) and long chain fatty acids (e.g., C₁₈ or greater), triglycerides such as caprylic/capric triglycerides (e.g., Miglyol; 812 available from Sasol Germany GmbH of Witten, Germany, shea butter (Butyrospermum Parkii), beeswax (e.g., White Beeswax SP-422P available from Strahl and Pitsch, New York), insect waxes, sperm whale oil, lanolin, vegetable waxes such as canauba wax, jojoba oil, candelilla wax; mineral waxes such as paraffin wax; and synthetic waxes such as C30-C45 olefins and C30-C45 alkyl methicones (e.g., ST-Wax 30 available from Dow Corning of Midland, Michigan); dicaprylyl carbonate (available as CETIOL CC from Cognis Corporation of Ambler, Pennsylvania); cetyl palmitate, lauryl palmitate, cetostearyl stearate, and polyethylene wax; and silicone waxes such as C30-45 alkyl methicone and C30-45 olefin (e.g., Dow Corning AMS-C30, having a melting point of 70°C, available from Dow Corning of Midland, Michigan).

The total concentration of the hydrophobic agents in the composition of the invention is preferably from about 1% to about 20%, preferably from about 2% to about 15%, more preferably from about 5% to about 15% by weight.

Compositions of the present invention further include one or more non-silicone, primary emulsifiers. By "one or more non-silicone, primary emulsifiers," it is meant one or more emulsifiers that do not include any siloxane linkages, whose total concentration is greater than the total concentration of silicone emulsifier in the composition. The non-silicone, primary emulsifiers generally comprise hydrogen and carbon atoms as well as optional oxygen, nitrogen, sulfur, and/or phosphorus atoms.

Examples of non-silicone, primary emulsifiers include fatty alcohols such as cetyl alcohol, stearyl alcohol and the like; esters of fatty alcohols such as: glycerol esters including glyceryl strearatc; polyethylene glycol ethers of fatty acid esters such as PEG-100 stearate; alkoxylated alcohols and polyglucosides. The non-silicone, primary emulsifier may comprise a blend of a high HLB (e.g., >15) fatty acid esters and a low HLB (e.g., <5) fatty acid ester. Suitably, the one or more non-silicone, primary emulsifiers may include a blend of a high HLB emulsifier (HLB> 15, such as PEG-100 stearate) and a low HLB emulsifier (HLB<5, such as glyceryl stearate). The melting point of the non-silicone, primary emulsifier may be greater than 30°C, such as greater than 50°C.

The one or more non-silicone, primary emulsifiers are generally capable of functioning as oil-in-water emulsifiers. As such, when combined with mineral oil and water (if there are multiple non-silicone, primary emulsifiers then they are combined in the same ratio present in the inventive composition), such that the total concentration of the non-silicone, primary emulsifiers (on an active basis) is 1 % by weight, the concentration of deionized water is about 96% by weight and the relative concentration of mineral oil is 3% by weight, the mixture can be agitated such that the mixture forms a stable emulsion of mineral oil in water that remains stable (no visible phase separation) when held at a temperature of about 25°C for at least 24 hours.

The total concentration of the non-silicone, primary emulsifiers in the composition of the invention is preferably from about 2% to about 10%, preferably from about 2% to about 8%, more preferably from about 3% to about 8% by weight.

The compositions of the present invention may preferably comprise a ratio of silicone water-in-oil emulsifier to non-silicone primary emulsifier (silicone water-in-oil emuisifier:non-silicone primary emulsifier) of from about 1:5 to about 1:12. More preferably, the compositions may comprise a ratio of about 1:5 to about 1:8, and even more preferably about 1:5 to about 1:6 water-in-oil emulsifier: non-silicone primary emulsifier.

Compositions of the present invention further comprise water. The concentration of water in the composition may range from about 40% to about 85%, preferably from about 45% to about 85%, more preferably from about 50% to about 85%, and even more preferably from about 60% to about 80%.

Compositions of the present invention preferably have solids contents that are at least about 15%, such as from about 15% to about 50%, preferably from about 15% to about 40%, more preferably from about 17% to about 35%, even more preferably from about 17% to about 30%. Solids content may be determined by means known to those skilled in the art, such as by placing a two gram sample of the composition in an aluminum tray and holding the sample at 120°C in a moisture analyzer (e.g., a Halogen Moisture Anlayzer HB 43 from Mettler-Toledo of Columbus, Ohio) for about 15-20 minutes until the weight becomes constant. Final weight is divided by initial weight, which is expressed as a percentage. As will be readily apparent to one skilled in the art, solids content will include ingredients, such as oils that generally show little volatility at 120°C.

Examples of functional ingredients typically used in personal care products, and in particular, so-called "leave-on" products, i.e., ones that are generally applied to the skin or hair and are left on for more than about 15 minutes before rinsing or washing. These optional ingredients include, for example, benefit agents, salts such as sodium chloride to enhance viscosity; dyes and colorants; opacificiers, matting agents, rheology modifiers; chelating and sequestering agents, pH adjusters, film forming polymers, fragrances; and preservatives (such as parabens, quaternary ammonium species, phenoxyethanol, benzoates, DMDM hydantoin), and thickening/gelling agents.

Examples of suitable benefit agents include those that provide benefits such as, but not limited to: depigmentation agents; reflectants and optical modifiers; amino acids and their derivatives; antimicrobial agents; allergy inhibitors; anti-acne agents; anti-aging agents; anti-wrinkling agents, antiseptics; analgesics; shine-control agents; antipruritics; local anesthetics; anti-hair loss agents; hair growth promoting agents; hair growth inhibitor agents, antihistamines; antiinfectives; anti-inflammatory agents; anticholinergics; vasoconstrictors; vasodilators; wound healing promoters; peptides, polypeptides and proteins; deodorants and anti-perspirants; medicament agents; skin firming agents, vitamins; skin lightening agents; skin darkening agents; antifungals; depilating agents; counterirritants; hemorrhoidals; insecticide; enzymes for exfoliation or other functional benefits; enzyme inhibitors; poison ivy products; poison oak products; burn products; anti-diaper rash agents; prickly heat agents; vitamins; herbal extracts; vitamin A and its derivatives; flavenoids; sensates and stress-reducing agents; anti-oxidants; hair lighteners; ultraviolet filters and other sunscreens; anti-edema agents, neo-collagen enhancers, anti-dandruff/sebhorreic dermatitis/psoriasis agents; keratolytics; lubricants; lightening and whitening agents; calcification, fluoridation and mineralization agents; and mixtures thereof.

The composition of the present invention may optionally further comprise one or more benefit agents such as, but not limited to, those agents listed above. Suitably, the composition of the present invention further comprising one or more benefit agents may be for use in medicine. More suitably, the composition of the present invention further comprising one or more benefit agents is for use in treating or preventing; signs of aging, acne, pigment contrast and pigmentation conditions, inflammation, redness, edema, dark circles, among other indications of the skin or muscosa.

Suitably, the composition of the present invention may further comprise an anti-acne agent for use in a method of treating acne. Optionally, the composition of the present invention may further comprise an anti-aging agent for use in a method of treating signs of aging. Suitably, the composition of the present invention may further comprise an anti-inflammatory agent for use in a method of treating inflammation. Suitably, the composition of the present invention may further comprise skin lightening agents or skin darkening agents and/or depigmentation agents for use in a method of treating pigment contrast and pigmentation conditions. The composition of the present invention may further comprise an anti-edema agent for use in a method of treating edema.

The compositions of the present invention may be made to be substantially free of certain ingredients. By "substantially free of it is meant that the composition has about 2% or less by weight of the particular ingredient, preferably about 1% or less, more preferably about 0.5% or less, more preferably about 0.1% or less, and most preferably the composition is free of the particular ingredient.

For example, the compositions of the present invention may be substantially free of non-emulsifylng silicone elastomers. By "non-emulsifying silicone elastomers," it is meant crosslinked siloxane material that are generally solid at room temperature and are not amphiphilic and/or do not meet the criteria set forth above for oil-in-water or water-in-oil emulsifiers. Examples of non-emulsifying silicone elastomers include crosspolymers of dimethicone and vinyl dimethicone (e.g., Dimethicone/Vinyl Dimethicone Crosspolymers sold as KSG 1610 and USG 107A, both from Shin-Etsu of Japan), among other non-emulsifying silicone elastomers.

Furthermore, by reducing or eliminating foaming surfactants such as those typically used in personal care cleansing products (typically for detergency and foaming), one can formulate a lotion that can has the aesthetic attributes suitable, for example, a skin care lotion, or even a hair conditioner. As such, the composition may have a "Maximum Foam "Volume" as determined by the "Foam Test" described below that is less than about 200 mL, preferably less than about 100 mL, more preferably less than about 50 mL, and even more preferably less than about 10 mL.

The following Foam Test is suitable to be performed on various personal care compositions to determine the Maximum Foam Volume upon agitation according to the present invention. The procedure is accomplished by adding 0.36 grams calcium chloride and 5.0 grams of the test product to 994.64 grams of deionized water and mixing until homogenous. The mixture is then added to a sample tank of a Sita R-2000 foam tester (commercially available from Future Digital Scientific, Co.; Bethpage, NY). The test parameters are set to repeat three runs (series count=3) of 250 ml sample size (fill volume=250 ml) with nine stir cycles (stir count=9) for a 30 second stir time per cycle (stir time=30 seconds) with the rotor spinning at 1300 RPM (revolution=1300) at a temperature setting of 30°C ±2°C. Foam Volume data is collected at each stir cycle and the average and standard deviation of the three runs is determined. Maximum Foam Volume is reported for each Example as the value after the ninth stir cycle.

Compositions of the present invention are typically extrudable or dispensable from a package, such as to be applied directly or indirectly, topically or orally to a body surface (e.g., skin, hair or mucosa). Depending upon the particular function, compositions of present invention are desirably rubbed onto the body surface and allowed to remain without rinsing.

Particularly suitable uses for compositions of the present invention include skin lotions, skin conditioners, and hair conditioners. Compositions of the present invention may also be used for oral care (e.g., toothpastes), personal lubricants, among other personal care applications.

The present invention provides methods oftreating a body surface of the human body comprising contacting at least a portion of the body with a composition of the present invention. Certain preferred methods comprising contacting a body surface with a composition of the present invention to condition, moisturize, treat or prevent: signs of aging, acne, pigment contrast and pigmentation conditions, inflammation, redness, edema, dark circles, among other indications of the skin or muscosa; as well as caries prevention, plaque control and other indications of the teeth or oral cavity.

### EXAMPLES

### Example I: Preparation of Inventive Examples

The inventive compositions of Example Ex.1 was prepared by first blending a particular ingredient with other ingredients according to the materials and amounts listed in Table 1:

**Table 1**

| | **Trade Name** | **Chemical Name** | **Weight Percent** |
|---|---|---|---|
| (1) | Deionized Water | Water | 73.483 |
| (2) | Emery 917 | Glycerin | 5.000 |
| (3) | Carbopol ETD 2020 | Acrylate (and) C10-30 Alkyl Acrylate Crosspolymer | 0.040 |
| (4) | Deionized Water | Sodium hydroxide solution (1.57%) | 0.127 |
| (5) | Sodium Hydroxide NF | | |
| (6) | Nipagin M | Methylparaben | 0.200 |
| (7) | Phenoxyethanol | Phenoxyethanol | 0.700 |
| (8) | Crodacol C95 | Cetearyl Alcohol | 3.000 |
| (9) | Miglyol 812 | Caprylic/Capric Triglyceride | 2.500 |
| (10) | Dow Corning 200 Fluid, 100CST | Dow Corning 200 Fluid, 100CST | 0.500 |
| (11) | Simulsol 165V | Glyceryl Stearate (and) PEG-100 Stearate | 2.000 |
| (12) | Cetiol SB 45 | Butyrospermum Parkii | 0.500 |
| (13) | Nipasol M | Propylparaben | 0.150 |
| (14) | Dow Corning 245 Fluid | Cyclopentasiloxane | 5.000 |
| (15) | Dow Corning 1413 Fluid | Dimethicone | 2.500 |
| (16) | Simulgel EG | Aqua (and) Isohexadecane (and) Polysorbate 80 (and) Sodium Acrylate (and) Acryloyldimethyl Taurate Copolymer | 0.800 |
| (17) | Silicone Water-In-Oil Emulsifier | | 2.5 |
| (18) | Amino-functional silicone gum | | 1.0 |
| | Total | | 100.000 |

The following process was used to prepare the inventive examples. An oil phase was prepared by combining CRODACOL (8) (a non-silicone, primary emulsifier), Miglyol 812 (9) (a hydrophobic agent), DC 200 Fluid (10) (a hydrophobic agent), Simulsol 165V (11) (a non-silicone, primary emulsifier), and Cetiol SB 45 (12) (a hydrophobic agent) in a beaker. The mixture was heated to 75°C, and mixed until completely dissolved. Propylparaben (13) (a preservative) was added and stirred for 10 minutes. A sodium hydroxide (pH adjuster) premix was prepared by mixing water (4) and sodium hyrdoxide pellets (5) and mixing until a clear solution was obtained. Main Tank - A water phase was prepared in the main vessel by combining DI Water (1) and glycerin (2) and stirring for 5 minutes. Carbopol (3) (a thickener) was then dispersed and added to the main vessel and mixed until completely dissolved. The water phase was heated to 70°C and the sodium hydroxide premix was added. Methyl paraben (6) and phenoxyethanol (7) (preservatives) were then added. The temperature of both oil phase and water phase were each maintained at 70-75°C.
The oil phase was then added to the water phase, while holding at 70-75°C, and mixed for about 20 minutes. The batch was slowly cooled to 48-52°C. DC1413 fluid (15) and DC 245 Fluid (14) (hydrophobic agents) and Simulgel EG (16) (a thicknener) were then added to the mixture. The mixture was then cooled to 35 - 40°C. Then the silicone emulsifiers (17) and amino gum (18) were added and mixed until homogenous.

Inventive Examples, Ex. 1-Ex.12 used various combinations of silicone water-in-oil emulsifier and amino-functional silicone gum (each as described above and listed by their trade names throughout the Examples), as shown below in Table 2:

**Table 2**

| **Example** | **Silicone Water-in-Oil Emulsifier** | **Low-Nitrogen Content Amino-Functional Silicone Gum** |
|---|---|---|
| Ex. 1 | ABIL EM97 | KF 8018 |
| Ex. 2 | ABIL EM90 | KF 8018 |
| Ex. 3 | KSG210 | KF 8018 |
| Ex. 4 | KF 6028 | KF 8018 |
| Ex. 5 | KF 6038 | KF 8018 |
| Ex. 6 | KF 6105 | KF 8018 |
| Ex. 7 | ABIL EM97 | SILAMINE Di-50-M |
| Ex. 8 | ABIL EM90 | SILAMINE Di-50-M |
| Ex. 9 | KSG 210 | SILAMINE Di-50-M |
| Ex. 10 | KF 6028 | SILAMINE Di-50-M |
| Ex. 11 | KF 6038 | SILAMINE Di-50-M |
| Ex. 12 | KF 6105 | SILAMINE Di-50-M |

### Example II: Preparation of Comparative Examples

The comparative Example Comp.1 was prepared by blending a particular ingredient with other ingredients. The concentrations of ingredients and process for making were identical to those described above for Inventive Ex. 1-12, except that either (1) the silicone emulsifier was not a water-in-oil emulsifier and/or (2) the amino-functional silicone gum did not have a low nitrogen content. As used herein and throughout the Examples, SILAMINE D2EDA is the tradename for polydimethylsiloxane amine available from Siltech Corp. which has a nitrogen content of greater than 4%. SILQUAT J2 is the tradename for quarternary polydimethylsiloxane available from Siltech Corp. which has a nitrogen content of greater than 3%. KF6100 is the tradename for Polyglyceryl-3 Disiloxane Dimethicone. KF6011 is the tradename for PEG-11 Methyl Ether Dimethicone.

**Table 2**

| **Example** | **Silicone Emulsifier** | **Amino-Functional Silicone Gum** |
|---|---|---|
| C1 | KF 6100* | SILAMINE D2EDA** |
| C2 | KF 6011* | SILAMINE D2EDA** |
| C3 | KF 6100* | SILQUAT J2** |
| C4 | KF 6011* | SILQUAT J2** |
| C5 | ABIL EM97 | SILAMINE D2EDA** |
| C6 | ABIL EM90 | SILAMINE D2EDA** |
| C7 | KSG 210 | SILAMINE D2EDA** |
| C8 | KF 6028 | SILAMINE D2EDA** |
| C9 | KF 6038 | SILAMINE D2EDA** |
| C10 | KF 6105 | SILAMINE D2EDA** |
| C11 | ABIL EM97 | SILQUAT J2** |
| C12 | ABIL EM90 | SILQUAT J2** |
| C13 | KSG 210 | SILQUAT J2** |
| C14 | KF 6028 | SILQUAT J2** |
| C15 | KF 6038 | SILQUAT J2** |
| C16 | KF 6105 | SILQUAT J2** |
| C17 | KF 6100* | KF 8018 |
| C18 | KF 6011* | KF 8018 |
| C19 | KF 6100* | SILAMINE Di-50-M |
| C20 | KF 6011* | SILAMINE Di-50-M |

| | | |
|---|---|---|
| * not water-in-oil ** not low nitrogen content | | |

### Example III: Preparation of comparative Examples

The comparative Example Comp.1 was prepared by blending a particular ingredient with other ingredients. The concentrations of ingredients and process for making were identical to those described above for Inventive Ex. 1-12, except that either a silicone emulsifier or amino-functional silicone gum was used, but not both. The amount of water used was adjusted (q.s.) so that all other concentrations were identical to those shown in Table 1.

**Table 3**

| **Example** | **Silicone Emulsifier** | **Amino-Functional Silicone Gum** |
|---|---|---|
| C21 | None | SILAMINE D2EDA** |
| C22 | None | SILQUAT J2** |
| C23 | None | KF 8018 |
| C24 | None | SILAMINE Di-50-M |
| C25 | KF 6100* | None |
| C26 | KF 6011* | None |
| C27 | ABIL EM97 | None |
| C28 | ABIL EM90 | None |
| C29 | KSG 210 | None |
| C30 | KF 6028 | None |
| C31 | KF 6038 | None |
| C32 | KF 6105 | None |

| | | |
|---|---|---|
| * not water-in-oil ** not low nitrogen content | | |

### Example IV: Evaluation of Structures of Inventive Examples and Comparative Examples

Inventive Examples Ex. 1-12 and Comparative Examples C1-C32 were evaluated for the presence and spacing of structures such as lamella or vesicles using Small Angle X-Ray Scattering (SAXS). A Hecus XRS Series I SAXS System was employed (available from Hecus X-Ray Systems GMBH of Graz, Austria). The SAXS system also included a Hiltonbrooks cabinet and generator, a Philips X-ray tube and tower, a Hecus modified Kratky camera system and a M Braun PSD and sample stage.

Samples were injected into a 1mm diameter quartz capillary (with a wall thickness of ~0.01mm), the capillary being held in a cuvette. The cuvette was then placed in a temperature controlled sample stage, itself being mounted within a compact Kratky camera, which was subsequently evacuated (~0.4x10-1 mbar). The temperature was maintained at 20°C. A beam of Cu Kalpha X-rays (wavelength=1.514A, generated at 40kV 30mA) was passed through a collimation system that produced a horizontal X-ray beam about 40x0.5mm which then traveled through the sample. Scatter from the sample was detected using a vertically mounted, gas-filled position sensitive detector, a 0.5mm Ni filter being used to attenuate the primary beam. The scatter signal was collected over a period of about 600 seconds which gives a good signal-to-noise ratio.

The scans of each of Inventive Examples Ex. 1-12 and Comparative Examples C1-C32 were consistent with the presence of structures having LC structures. In certain cases, only short-range order was present. In certain other cases, both short and long range order were present. For those samples showing short and long range order, two numbers are shown in Table 4 below:

**Table 4**

| **Example** | **Spacing (Angstroms)** | **Ordering** |
|---|---|---|
| Ex. 1 | | None |
| Ex. 2 | 254, 50 | Long and Short |
| Ex. 3 | 254, 50 | Long and Short |
| Ex. 4 | | None |
| Ex. 5 | 254, 50 | Long and Short |
| Ex. 6 | 254, 50 | Long and Short |
| Ex. 7 | 254, 50 | Long and Short |
| Ex. 8 | 254, 50 | Long and Short |
| Ex. 9 | | None |
| Ex. 10 | 254, 50 | Long and Short |
| Ex. 11 | 254, 50 | Long and Short |
| Ex. 12 | 254, 50 | Long and Short |
| C1 | 50 | Short |
| C2 | 50 | Short |
| C3 | 50 | Short |
| C4 | 50 | Short |
| C5 | 50 | Short |
| C6 | 50 | Short |
| C7 | | None |
| C8 | | None |
| C9 | 50 | Short |
| C10 | 50 | Short |
| C11 | | None |
| C12 | | None |
| C13 | | None |
| C14 | | None |
| C15 | 50 | Short |
| C16 | | None |
| C17 | 37 | Short |
| C18 | 37 | Short |
| C19 | 37 | Short |
| C20 | | None |
| C21 | 50,37 | Short |
| C22 | 50, 37 | Short |
| C23 | 231, 50 | Long and Short |
| C24 | 254,50 | Long and Short |
| C25 | | None |
| C26 | 37 | Short |
| C27 | 254, 50 | Long and Short |
| C28 | 254,50 | Long and Short |
| C29 | 254, 50 | Long and Short |
| C30 | 254, 50 | Long and Short |
| C31 | 254,50 | Long and Short |
| C32 | 254,50 | Long and Short |

The results of the SAXS testing indicate that Comparative Examples C23-C24 and C27-C32 (which include either a W/O silicone emulsifier or a low nitrogen content amino-functional silicone gum, but not both) show both long range and short range ordering. The remaining comparative examples, which have neither a low nitrogen content amino-functional silicone gum nor a water-in-oil silicone emulsifier show only short range ordering or no ordering. However, surprisingly nine of the twelve of the inventive examples (which have a W/O silicone emulsifier and a low nitrogen content amino-functional silicone gum) have both long and short range ordering. These results suggest that by including both a W/O silicone emulsifier and a low nitrogen content amino-functional silicone gum one surprisingly tends to preserve the presence of both long range ordered and short range ordered LC structures.

### Example V: Evaluation of Tactile Aesthetics

Four formulations were evaluated for tactile aesthetics: Inventive Example Ex. 5 (which included a low nitrogen content amino-functional silicone gum and a water-in-oil silicone emulsifier; and possessed long and short range order); Comparative Example C23 (which included the low nitrogen content amino-functional silicone gum but not the water-in-oil silicone emulsifier, and possessed long and short range order); Comparative Example C3 (which included the water-in-oil silicone emulsifier but not the low nitrogen content amino-functional silicone gum; and possessed long and short range order); and an additional Comparative Example, C33. Comparative Example, C33 was identical to Comparative Example C23, except that it included 2.5% of a silicone elastomer, Dow Corning 9566 Elastomer Blend, available from Dow Corning, in place of the aminogum.

The compositions were each evaluated for pickup. By "pickup," it is meant the amount of product that remains on the finger of the panelist after touching the product. The test was conducted in the following manner: compositions were available in separate ½ oz. low profile containers. The surface of the product was smoothed using a tongue depressor. The evaluation was done when the hands of the panelist were washed, but not cleaned with alcohol. Panelists were instructed to position the container at eye level, close their eyes, and using the index finger of their dominant hand, push through the product until the finger was half submerged. Then open eyes, steady the container with the free hand and slowly pull the submerged finger straight up out of the product. When the finger is away from the product, evaluate the relative amount of product on the finger. Each panelist evaluated the compositions and rated them for amount of pickup on a scale from 1 to 10 with 10 being the most pickup. The means of the pickup scores as assigned by panelist are reported in Table 5.

**Table 5**

| **Example** | **Includes** | **Mean "Pickup" Score*** |
|---|---|---|
| E5 | low nitrogen content amino-functional silicone gum and a W/O silicone emulsifier | 6.0 |
| C23 | low nitrogen content amino-functional silicone gum | 5.6 |
| C31 | W/O silicone emulsifier | 4.9 |
| C33 | Non-emulsifying silicone | 6.6 |

| | | |
|---|---|---|
| * The score for E5 was significantly higher than C23 and C31 at a 99% confidence level. | | |

The results of the tactile aesthetics testing indicate that surprisingly, Inventive Example E5, which has both the low nitrogen content amino-functional silicone gum and a W/O silicone emulsifier scores substantially higher with respect to tactile aesthetic "pickup" than the comparative formulas which had either the low nitrogen content amino-functional silicone gum or a W/O silicone emulsifier, but not both.

## Claims

1. A composition comprising a silicone water-in-oil emulsifier, an amino-functional silicone gum having a nitrogen content that is about 3% or less by weight based on the total weight of amino-functional silicone gum, a hydrophobic agent, a non-silicone primary emulsifier, and water.

2. The composition of claim 1 wherein the silicone water-in-oil emulsifier comprises a non-crosslinked dimethicone copolyol.

3. The composition of claim 2 wherein the silicone water-in-oil emulsifier is selected from the group consisting of cetyl dimethicone copolyols, branched polyether and alkyl modified silicones, branched polyglycerin and alkyl modified silicones, and combinations of two or more thereof.

4. The composition of claim 1 wherein the silicone water-in-oil emulsifier comprises a crosslinked elastomeric solid organopolysiloxane.

5. The composition of claim 4 wherein said crosslinked elastomeric solid organopolysiloxane is selected from the group consisting of polyether-modified crosslinked siloxanes, polyglycerin-modified crosslinked siloxanes, and combinations of two or more thereof.

6. The composition of any preceding claim wherein said amino-functional silicone gum comprises a gum selected from the group consisting of aminoalkyldimethicones, morpholine-modified polydimethylsiloxanes, diethanolamine-modified polydimethylsiloxanes, and combinations of two or more thereof.

7. The composition of any preceding claim wherein the non-silicone primary emulsifier comprises a material selected from the group consisting of fatty alcohols, esters of fatty alcohols, polyethylene glycol ethers of fatty acid esters, alkoxylated alcohols and polyglucosides, and combinations of two or more thereof.

8. The composition of any preceding claim wherein the non-silicone primary emulsifier comprises a material selected from the group consisting of cetyl alcohol, stearyl alcohol, glyceryl stearate, PEG-100 stearate, and combinations of two or more thereof.

9. The composition of any preceding claim, wherein the composition has a solids content of about 15% by weight or greater.

10. The composition of any preceding claim, wherein the composition has a solids content of from about 15% by weight to about 50% by weight.

11. The composition of any preceding claim, wherein the silicone water-in-oil emulsifier is present in a concentration from about 0.3% by weight to about 5% by weight.

12. The composition of any preceding claim, wherein the amino-functional silicone gum is present in a concentration from about 0.5% by weight to about 5% by weight.

13. The composition of any preceding claim, wherein the water is present in a concentration from about 40% by weight to about 85% by weight.

14. The composition of any preceding claim, wherein the hydrophobic agent is present in a concentration from about 2% by weight to about 15% by weight.

15. The composition of any preceding claim, wherein the composition is substantially-free of non-emulsifying silicone elastomers.

16. The composition of any preceding claim, wherein the ratio of silicone water-in-oil emulsifier to non-silicone primary emulsifier is from about 1:5 to about 1:12.

17. A composition comprising from about 0.3% to about 5% by weight of a silicone oil-in-water emulsifier, from about 0.5% to about 5% by weight of an amino-functional silicone gum having a nitrogen content that is about 3% or less by weight based on the total weight of the amino-functional silicone gum, a hydrophobic agent, a non-silicone primary emulsifier, and water, wherein the composition comprises about 0.5% or less of non-emulsifying silicone elastomers.

18. The composition of claim 17 wherein said silicone oil-in-water emulsifier comprises a material selected from the group consisting of cetyl dimethicone copolyols, branched polyether and alkyl modified silicones, branched polyglycerin and alkyl modified silicones, polyether-modified crosslinked siloxanes, polyglycerin-modified crosslinked siloxanes, and combinations of two or more thereof, and said amino-functional silicone gum comprises a material selected from the group consisting of aminoalkyldimethicones, morpholine-modified polydimethylsiloxanes, diethanolamine-modified polydimethylsiloxanes, and combinations of two or more thereof.

19. Use of a composition according to any preceding claim in personal care products.
